(19) Europäisches Patentamt
European Patent Office
Office européen des brevets

(11) EP 0 482 183 B1

(12) FASCICULE DE BREVET EUROPEEN

(45) Date de publication et mention de la délivrance du brevet: **30.07.1997 Bulletin 1997/31**

(21) Numéro de dépôt: **91910312.7**

(22) Date de dépôt: **10.05.1991**

(51) Int Cl.[6]: **C07D 233/54**, A61K 31/415

(86) Numéro de dépôt international: **PCT/FR91/00384**

(87) Numéro de publication internationale: **WO 91/17146 (14.11.1991 Gazette 1991/26)**

# (54) COMPOSES AGONISTES DU RECEPTEUR H3 DE L'HISTAMINE A USAGE THERAPEUTIQUE, COMPOSITIONS PHARMACEUTIQUES AGISSANT COMME AGONISTES DUDIT RECEPTEUR ET PROCEDE DE PREPARATION

HISTAMIN-H3-REZEPTOR-AGONISTEN ZUR THERAPEUTISCHEN ANWENDUNG, PHARMAZEUTISCHE ZUBEREITUNGEN, DIE AN H3-REZEPTOREN-AGONISTISCH WIRKEN, UN DIE METHODE IHRER DARSTELLUNG

HISTAMINE H3-RECEPTOR AGONIST COMPOUNDS FOR THERAPEUTIC USE, PHARMACEUTICAL COMPOSITIONS ACTING AS AGONISTS OF SAID RECEPTOR AND METHOD OF PREPARATION

(84) Etats contractants désignés:
**AT BE CH DE DK ES FR GB GR IT LI LU NL SE**

(30) Priorité: **09.05.1990 FR 9005776**

(43) Date de publication de la demande:
**29.04.1992 Bulletin 1992/18**

(73) Titulaires:
- **INSTITUT NATIONAL DE LA SANTE ET DE LA RECHERCHE MEDICALE (INSERM)**
  **F-75654 Paris Cédex 13 (FR)**
- **SOCIETE CIVILE BIOPROJET**
  **F-75003 Paris (FR)**

(72) Inventeurs:
- **GARBARG, Monique**
  **F-75017 Paris (FR)**
- **ARRANG, Jean-Michel**
  **160 avenue du Général-Leclerc**
  **F-91190 Gif-sur-Yvette (FR)**
- **SCHUNACK, Walter**
  **D-1000 Berlin 38 (DE)**
- **LIPP, Ralph**
  **D-1000 Berlin 30 (DE)**
- **STARK, Holger**
  **D-1000 Berlin 30 (DE)**
- **LECOMTE, Jeanne-Marie**
  **F-75003 Paris (FR)**
- **SCHWARTZ, Jean-Charles**
  **F-75014 Paris (FR)**

(74) Mandataire: **Bernasconi, Jean et al**
**c/o Cabinet Lavoix,**
**2, Place d'Estienne d'Orves**
**75441 Paris Cédex (FR)**

(56) Documents cités:
**EP-A- 0 024 639** **EP-A- 0 214 058**
**FR-A- 2 548 183**

- **Chemical Abstracts, vol. 100, no. 25, 18 juin 1984, (Columbus, Ohio, US), voir page 545, résumé 209240f, & JP, A, 5916867 (NIPPON CHEMIPHAR. CO., LTD) 28 janvier 1984**
- **Journal of the American Chemical Society, vol. 99, no. 3, 2 février 1977, H.E. Smith et al.: "Optically active amines. 22. Application of the salicylidenimino chirality rule to alpha-amino acids", pages 707-713**
- **Journal of Organic Chemistry, vol. 48, 1983, (US), J. Sunamoto et al.: "Intramolecular cyclization of the pyridoxal-histidine Schiff base controlled in reversed micelles", pages 2423,2424,**
- **Chemical and Pharmaceutical Bulletin, vol. 12, no. 3, 1964, A. Inoue et al.: "On the reaction products between dehydroacetic acid and amino acids", pages 382,383,**
- **Chromatographia, vol. 23, no. 9, septembre 1987, K. Jinno et al.: "Retention prediction of o-phthalaldehyde amino acid derivatives in reversed-phase liquid chromatography", pages 675-679**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

- **Journal of the Chemical Society C, no. 4, 1968, A.F. Al-Sayyab et al.: "Schiff bases. Part I. Thermal decarboxylation of alpha-amino-acids in the presence of ketones", pages 406-410,**
- **Journal of Protein Chemistry, vol. 7, no. 5, 1988, M. Maekelae et al.: "Acid- base chemistry of vitamin B6 compounds in methanol", pages 549-559**
- **Journal of Organic Chemistry, vol. 47, 1982, (US), M.J. O'Donnell et al.: "A mild and efficient route to Schiff base derivatives of amino acids", pages 2663-2666**
- **Chemical Abstracts, vol. 95, no. 3, 20 juillet 1981, (Columbus, Ohio, US), L. Casella et al.: "Different coordination modes of histidine. Circular dichroism study of copper(II) complexes of the Schiff bases derived from (+)-hydroxymethylenecamphor and histidine derivatives", voir page 236, résumé 19963z, & Congr. Naz. Chim. Inorg. (Atti), 13th 1980, pages 227-229**
- **Liebigs Annalen der Chemie, no. 2, février 1982, H. Doepp et al.: "Die Konstitution der Musca-aurine", pages 254-264,**
- **Journal of the Chemical Society C, no. 22, 1966, Z. Badr et al.: "Optial rotatory dispersion. Part XXXVIII. Azomethines. Part III. Configurational assignment in the alpha-amino-acid series using N-alkylidene derivatives", pages 2047-2051**
- **Chimiko-Farmacevticeskij Zurnal, vol. 21, no. 9, 1987, S.Yu. Ostrovskii et al.: "Role of histidine in the nonenzymatic inactivation of acetaldehyde", pages 1034-1038**
- **Lipids, vol. 21, no. 1, 1986, V. Nair et al.: "The chemistry of lipid peroxidation metabolites: crosslinking reactions of malondialdehyde", pages 6- 10**


Remarques:

Le dossier contient des informations techniques présentées postérieurement au dépôt de la demande et ne figurant pas dans le présent fascicule.

## Description

L'invention concerne des comDosés notamment à usage thérapeutique comme agonistes du récepteur $H_3$ de l'histamine. des compositions pharmaceutiques les contenant et l'utilisation de ces composés pour la réalisation d'un médicament.

Dans les demandes de brevet EP-A-0 214 058 et EP-A-0 338 939, les intéressantes propriétés agonistes du récepteur histaminergique $H_3$ de dérivés de l'imidazoléthylamine ont été décrites. Ces composés sont très puissants in vitro puisqu'ils agissent à des concentrations nanomolaires. Ils sont également très actifs in vivo chez le rongeur (rat ou souris) sur lequel ils exercent une inhibition de la synthèse et de la libération d'histamine endogène du cerveau et de divers organes périphériques à des doses voisines de 1 mg/kg : par exemple, la dose efficace 50 % de la (R)α-méthylhistamine est de 3 mg/kg par voie orale (Arrang et al., Nature 1987, 327, 117-123 ; Garbarg et al., Eur. J. Pharmacol. 1989, 164, 1-11). Cette forte activité in vivo est confirmée par la mesure du taux plasmatique de la (R)α-méthylhistamine déterminé soit par un dosage radio-enzymatique (Garbarg et al., Eur. J. Pharmacol. 1989, 164, 1-11), soit par un dosage radio-immunologique: à une dose orale de 3 mg/kg, des taux plasmatiques suffisants pour stimuler le récepteur $H_3$ (>10 nM) sont maintenus pendant plus de 6 heures.

Or, en administrant le même composé à une série de volontaires sains à la dose de 175 mg par personne, les déposants ont constaté que les taux plasmatiques de (R)α-méthylhistamine qui résultaient de cette administration étaient environ 10 fois plus faibles que ceux que l'on attendait au vu des résultats correspondants obtenus chez le rongeur.

Les déposants ont découvert de façon surprenante qu'en bloquant la fonction amine primaire de dérivés de l'histamine, notamment parmi ceux décrits dans les susdites demandes de brevet européen EP-A-0 214 058 et EP-A-0 338 939, on obtient des composés extrêmement actifs in vivo qui sont lentement hydrolysables en milieu neutre pour redonner le dérivé libre. On obtient des résultats semblables avec l'ensemble des amines à fortes affinité et sélectivité pour le récepteur $H_3$ de l'histamine.

L'invention a donc pour objet un composé provenant d'une amine $R\text{-}NH_2$, R étant tel que ladite amine possède une forte affinité pour le récepteur $H_3$ de l'histamine, à la fonction amine primaire de laquelle est lié un groupe déterminant une liaison lentement hydrolysable en milieu neutre.

Par composé, on entend également les différentes formes isomériques ainsi que, le cas échéant, les dérivés obtenus par condensation d'eau comme il sera expliqué plus en détail par la suite.

Les amines $R\text{-}NH_2$ concernées par l'invention sont plus particulièrement les dérivés de l'histamine, ou plus précisément les amines de même formule chimique, décrits dans les susdites demandes de brevet EP-A-0 214 058 et EP-A-0 338 939, et qui répondent à la formule :

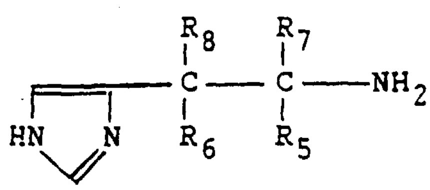

dans laquelle $R_5$, $R_6$ et $R_8$ désignent chacun un atome d'hydrogène ou un groupe méthyle, ou bien $R_5$ et $R_6$, pris ensemble, forment un groupe méthylène et $R_7$ représente un atome d'hydrogène ou un groupe méthyle, $R_5$, $R_6$, $R_7$ et $R_8$ ne pouvant désigner simultanément l'hydrogène.

Parmi les amines qui répondent à cette formule, on peut en particulier citer les suivantes :

1. α-méthylhistamine ou 4-(2-aminopropyl)-imidazole et plus particulièrement son stéréoisomère R(-) ; ($R_5 = CH_3$; $R_6 = R_7 = R_8 = H$) ; décrite par Gerhard et Schunack, Arc. Pharm. (Weinheim) 1980, 313, 709.
2. α,α-diméthylhistamine ou 4-(2-méthyl 2-amino propyl)-imidazole ($R_5 = R_7 = CH_3$ ; $R_6 = R_8 = H$) ; décrite par Schunack, Joint Meeting of the American Chemical Society, Div. of Med. Chem., and the American Society for pharmacology and experimental therapeutic, Boston, USA, 18-22 août, 1985.
3. β-méthylhistamine ou 4-(1-méthyl 2-amino éthyl)-imidazole et ses stéréoisomères ($R_6 = CH_3$ ; $R_5 = R_7 = R_8 = H$) décrite par Ganellin et al. dans J. Med. Chem. 1973, 16, 616 (sous forme racémique) et par Schunack et al. dans Frontiers in histamine research, C.R. Ganellin et J.C. Schwartz, éd. Pergamon Press, 1985, p. 39 (sous forme de stéréoisomères).
4. β,β-diméthylhistamine ou 4-(1,1-diméthyl 2-amino éthyl)-imidazole ($R_5 = R_7 = H$ ; $R_6 = R_8 = CH_3$), décrite par Durant et al., J. Med. Chem. 1976, 19, 923.
5. 2-(4-imidazolyl)-cyclopropylamine ($R_5 = R_6 = CH_2$; $R_7 = R_8 = H$), décrite par Burger et al., J. Med. Chem. 1970,

13, 33.

6. $\alpha,\beta$-diméthylhistamine et ses stéréoisomères ($R_5 = R_8 = CH_3$ ; $R_6 = R_7 = H$), décrite dans le EP-A-0 338 939.

Selon l'invention, les amines préférées sont notamment la (R)$\alpha$-méthylhistamine, l'$\alpha,\alpha$-diméthylhistamine et l'$\alpha,\beta$-diméthylhistamine.

Selon l'invention, le composé peut comprendre un résidu de cétone ou d'aldhéhyde, notamment de cétone ou d'aldéhyde cyclique, lié à la fonction amine primaire de ladite amine.

La présente invention a plus particulièrement pour objet un composé dérivant de l'histamine ayant une forte affinité pour le récepteur $H_3$ de l'histamine, caractérisé en ce que ledit composé provient d'un dérivé de l'histamine répondant à la formule générale

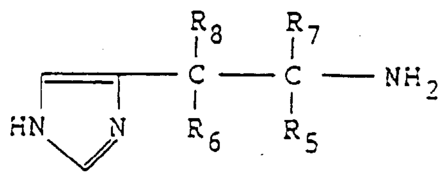

(2)

dans laquelle $R_5$, $R_6$ et $R_8$ désignent chacun un atome d'hydrogène ou un groupe méthyle ou bien $R_5$ et $R_6$, pris ensemble, forment un groupe méthylène et $R_7$ représente un atome d'hydrogène ou un groupe méthyle, $R_5$, $R_6$, $R_7$ et $R_8$ ne pouvant désigner simultanément l'hydrogène, à la fonction amine primaire duquel est lié un groupe déterminant une liaison lentement hydrolysable en milieu neutre.

Les composés préférés conformes à l'invention sont caractérisés en ce-qu'ils sont constitués par des azométhines répondant à la formule générale

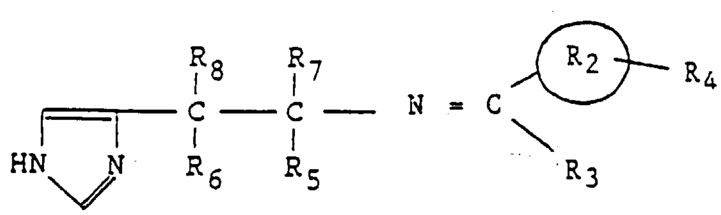

(3)

où $R_5$, $R_6$, $R_7$ et $R_8$ représente chacun un atome d'hydrogène ou un groupe méthyle ou bien $R_5$ et $R_6$, pris ensemble, forment un groupe méthylène, $R_5$, $R_6$, $R_7$ et $R_8$ ne pouvant désigner simultanément l'hydrogène,

$R_2$ est un groupe aryle ou un groupe hétérocyclique éventuellement lié à $R_3$ et éventuellement mono- ou polysubstitué avec $R_4$,
$R_3$ à la signification de $R_2$ ou $R_4$ et
$R_4$ est l'hydrogène ou $OR_3$ ou $COOR_3$ ou un halogène ou $CT_3$ ou un alkyle non substitué, mono- ou polysubstitué ou

ou par l'un de leurs sels, tels que, notamment, les chlorhydrates, maléates.

Les formules chimiques qui suivent représentent des composés préférés conformes à l'invention, cette liste n'étant, bien sur, pas limitative :

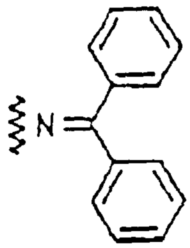 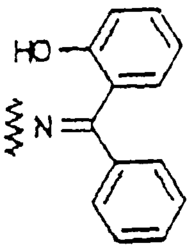 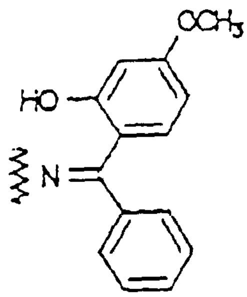

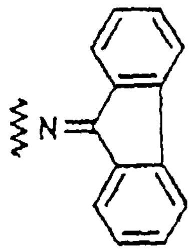 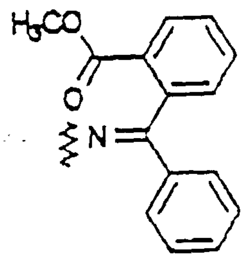 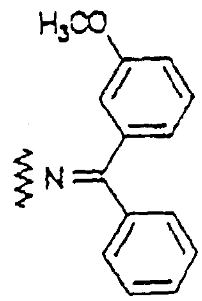

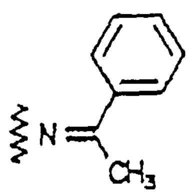 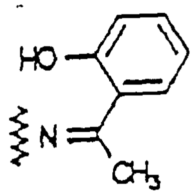 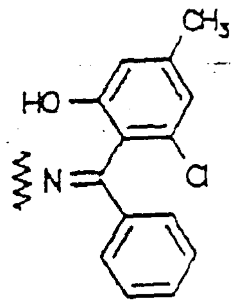

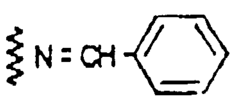 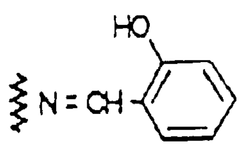 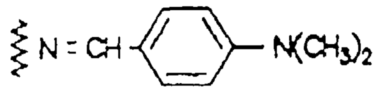

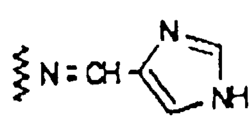

formules dans lesquelles N est l'atome d'azote de la fonction amine primaire de l'amine lié, dont le reste de la molécule n'est par représenté et qui peut être notamment l'un des deux dérivés de l'histamine suivants :

- (R)α-méthylhistamine

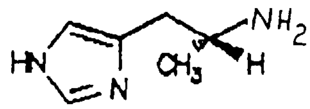

- α,α-diméthylhistamine

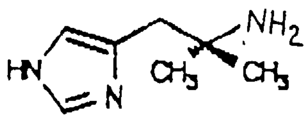

α,β-diméthylhistamine et plus particulièrement son isomère α-R, β-S.

Les composés selon l'invention sont de préférence réalisés par condensation d'une amine libre et d'un aldhéhyde ou d'une cétone cycliques, ce qui doit conduire à une base de Schiff lentement hydrolysable en milieu neutre.

Un exemple de synthèse est fourni ci-après:

Préparation du (R)-(-)-2-[N-[1-(1H-imidazol-4-yl)-2-propyl] -(iminophénylméthyl)]phénol (n° 94) à partir du (R)-α-MeHA dihydromaléate:

2,24 g (20 mmole) de tert. butoxyde de potassium sont dissous dans 30 ml de 2-propanol anhydre. Après addition de 1,79 g (5 mmole) de (R)-α-MeHA dihydromaléate, la suspension obtenue est agitée une nuit. La matière qui se sépare (maléate disodique) est séparée par filtration et lavée deux fois par 5 ml de 2-propanol anhydre. Les filtrats rassemblés sont évaporés à siccité et redissous dans 40 ml d' éthanol anhydre. Après addition de 1,19 g (6 mmole) de 2-hydroxybenzophénone, la solution est mise à reflux pendant 15 mn et évaporée à siccité. Du diéthyléther et quelques cristaux d'ensemencement sont ajoutés à l'huile obtenue et le mélange est agité pour la cristallisation. La matière jaune obtenue est recristallisée deux fois a partir de cyclohexane/acétate d' éthyle anhydre.

Rendement 63,7 % F: 142-144°C (cyclohexane/EtOAc)

| | | | | |
|---|---|---|---|---|
| $C_{19}H_{19}N_3O$ (305,4) | calculé | C 74,73 | H 6,27 | N 13,76 |
| | trouvé | C 74,43 | H 6,33 | N 13,79 |

RMN $^1H$: [D6]DMSO (300MHz) δ = 15,63 (s, remplaçable par $D_2O$, 1H, Im-NH), 11,73 (br, remplaçable par $D_2O$, 1H, OH), 5,51-6,54 (m, 11H, arom), 3,53 (dt, $J_1=J_2$=6Hz, 1H, CH-$CH_2$), 2,73 (d, J=6Hz, 2H, $CH_2$), 1,15 (d, J=6Hz, 3H, $CH_3$), ppm rel. TMS,

MS: EI m/z (rel.int.[%]) 305 ($[M]^+$, 6), 224 (43), 172 (72), 171 (44), 144 (34), 118 (51), 89 (40), 69 (86), 41 (100).

IR: 3076 m, 2966 m, 2600 ns, 1961 s, 1606 s, 1571 $cm^{-1}$. $[\alpha]_D^{20}$ = -295,4° (MeOH, c=1).

Dans certains cas, le composé obtenu peut lui-même se recombiner sous forme d'un dérivé, sans perdre ses propriétés, notamment son aptitude à l'hydrolyse et à la libération progressive de l'amine de départ, et l'on peut être en présence d'un mélange de différentes formes du composé en question.

L'invention concerne aussi de tels dérivés et des mélanges des différentes formes du composé, comme cela a été déjà dit plus haut.

La réaction suivante illustre ce phénomène dans le cas de la (R)α-méthylhistamine :

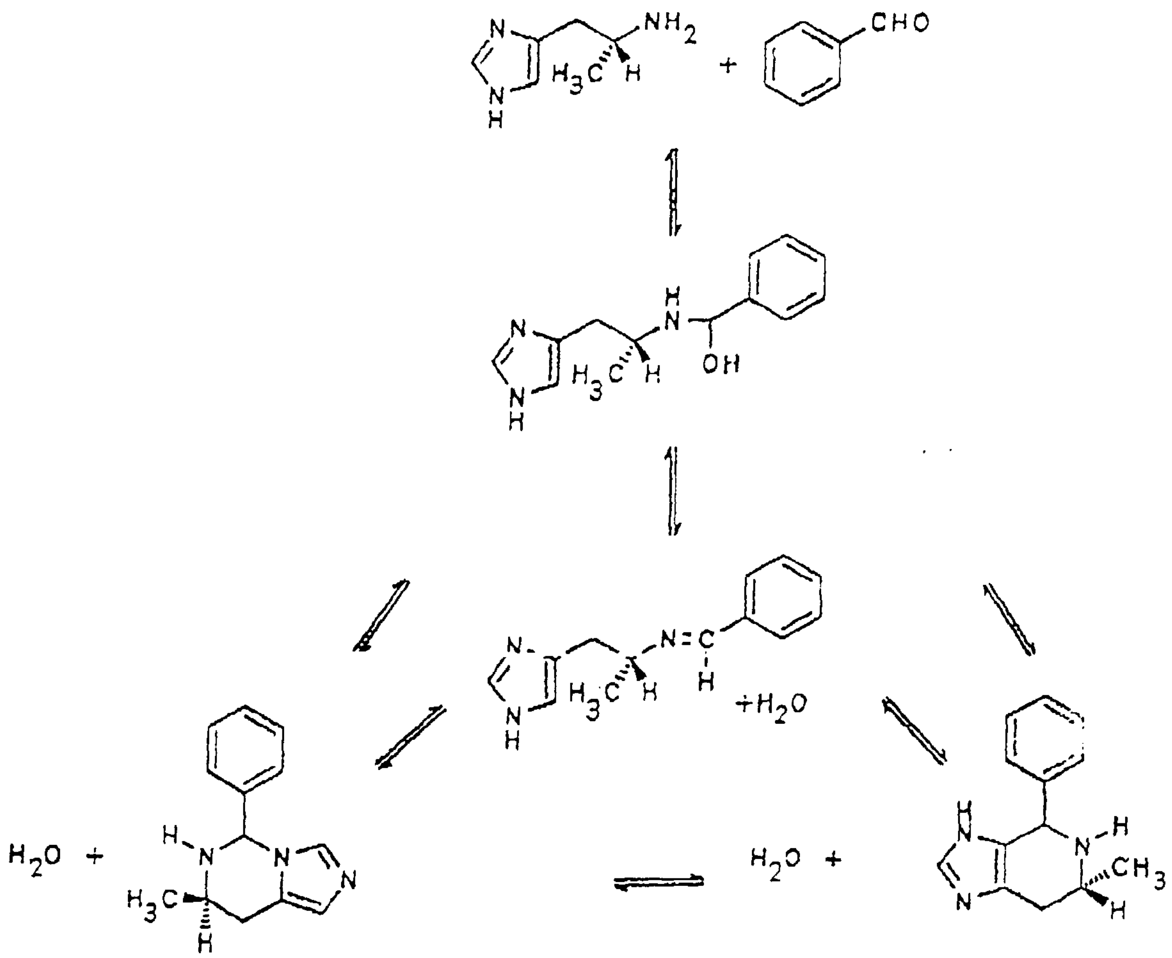

On pense que les médicaments ou compositions pharmaceutiaues selon l'invention agissent à la manière d'une prodroque se transformant lentement, dans l'organisme, en principe biochimiquement actif.

L'invention a encore pour objet une composition pharmaceutique comprenant un composé selon l'invention et un véhicule ou excipient pharmaceutiquement acceptable.

L'invention a également pour objet l'utilisation d'un composé de formule (1) et, de préférence, (2) ou (3) pour la réalisation d'un médicament inhibiteur de la synthèse et de la libération d'histamine ou d'autres médiateurs régulés par le récepteur $H_3$, notamment à effets sédatif, régulateur du sommeil, anticonvulsivant, antivertigineux, antidépresseur, antiallergique, anti-asthmatique, antiprurigineux anti-inflammatoire (y compris dans la sphère gastro-intestinale), antisécrétoire ou antiulcéreux.

D'autres avantages de l'invention vont ressortir de la description de composés selon l'invention, pris à titre d'exemples non limitatifs, et d'essais réalisés avec ces composés.

On a réalisé le composé I suivant par condensation de la (R)$\alpha$-méthylhistamine :

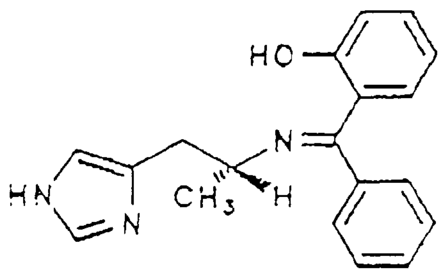

Il s'est avéré que :

1) le composé I est lentement hydrolysable in vitro en milieu neutre (t1/2 > 3 h à 25°C) en présence comme en l'absence d'extraits tissulaires ;
2) administré par voie orale au rongeur, il est bien absorbé comme en témoignent les taux plasmatiques déterminés par hydrolyse chimique suivie de dosages radio-immunologiques de la (R)α-méthylhistamine ainsi produite in vitro ;
3) le composé I est bien hydrolysé in vivo chez le rongeur car son administration conduit à des taux plasmatiques de (R)α-méthylhistamine au moins égaux à ceux que réalise l'administration d'une dose molaire identique de l'amine libre ; qui plus est, ces taux sont maintenus plus longtemps et il en résulte une baisse de synthèse d'histamine endogène plus accentuée (notamment dans le cerveau);
4) administré au chimpanzé et au volontaire sain humain, le composé I conduit à des taux plasmatiques de (R)α-méthylhistamine de 20 à 100 fois plus élevés qu'une dose molaire équivalente de l'amine libre ; qui plus est, des taux correspondants à une activation des récepteurs $H_3$ sont maintenus plus longtemps en raison de l'hydrolyse progressive du composé I (dont le taux plasmatique est toujours 5 à 10 fois plus élevé que celui de l'amine libre).

Ainsi, les doses actives chez l'homme par voie orale sont, de préférence, voisines de 1 à 20 mg au lieu de 100 à 300 mg pour l'amine libre. On peut constater une réduction de la toxicité et des effets secondaires et la baisse du coût de préparation d'une dose unitaire. Qui plus est, le composé I est nettement plus lipophile que l'amine libre, ce qui devrait faciliter le passage des barrières membranaires comme les barrières digestive ou hémato-encéphalique et l'atteinte de récepteurs $H_3$ normalement peu accessibles à une amine libre.

**Revendications**

1. Composé dérivant de l'histamine ayant une forte affinité pour le récepteur $H_3$ de l'histamine, caractérisé en ce qu'il est constitué par un azométhine de formule générale

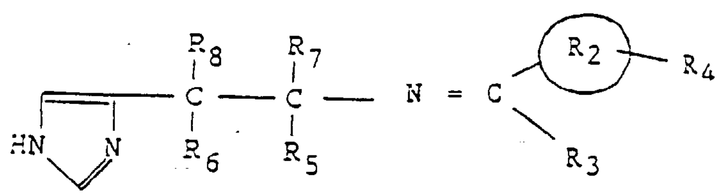

où $R_5$, $R_6$, $R_7$ et $R_8$ représentent chacun un atome d'hydrogène ou un groupe méthyle ou bien $R_5$ et $R_6$, pris ensemble, forment un groupe méthylène, $R_5$, $R_6$, $R_7$ et $R_8$ ne pouvant désigner simultanément l'hydrogène,

$R_2$ est un groupe aryle ou un groupe hétérocyclique éventuellement lié à $R_3$ et éventuellement mono- ou polysubstitué avec $R_4$,
$R_3$ a la signification de $R_2$ ou $R_4$ et
$R_4$ est l'hydrogène ou $OR_3$ ou $COOR_3$ ou un halogène ou $CF_3$ ou un alkyle non substitué, mono- ou polysubstitué ou l'un de ses sels pharmaceutiquement acceptables.

2. Composé selon la revendication 1, caractérisé en ce que $R_5$ est le méthyle et $R_6$, $R_7$ et $R_8$ représentent chacun un atome d'hydrogène.

3. Composé selon la revendication 1, caractérisé en ce que R5 et $R_7$ sont chacun le méthyle et $R_6$ et $R_7$ représentent chacun un atome d'hydrogène.

4. Composé selon la revendication 1, caractérisé en ce que $R_5$ et $R_8$ sont chacun le méthyle et $R_6$ et $R_7$ représentent chacun un atome d'hydrogène.

5. Composé selon la revendication 1, caractérisé en ce que $R_2$ est un groupe phényle éventuellement mono- ou polysubstitué par un radical $R_4$ représentant un hydroxy, un méthoxy , un halogène ou COO-$CH_3$ et $R_3$ représente

un groupe phényle.

6. Composé selon la revendication 1, caractérisé en ce que $R_2$ est un groupe phényle éventuellement mono- ou polysubstitué par un radical $R_4$ ou polysubstitué par un radical $R_4$ représentant un hydroxy, un méthyle, un halogène ou un diméthylamino, et $R_3$ représente un atome d'hydrogène ou le méthyle.

7. Composé selon la revendication 1, caractérisé en ce que $R_2$ représente un radical imidazolyle, $R_3$ et $R_4$ un atome d'hydrogène.

8. Utilisation du composé selon l'une quelconque des revendications 1 à 7, pour la préparation d'un médicament inhibiteur de la synthèse et de la libération d'histamine ou d'autres médiateurs régulés par le récepteur $H_3$, notamment à effets sédatif, régulateur du sommeil, anticonvulsivant, antivertigineux, antidépresseur, antiallergique, antiasmathique, antiprurigineux, anti-inflammatoire, antisécrétoire ou antiulcéreux.

9. Composition pharmaceutique destinée à inhiber la synthèse et la libération des médiateurs régulés par les récepteurs $H_3$ contenant un composé de formule générale

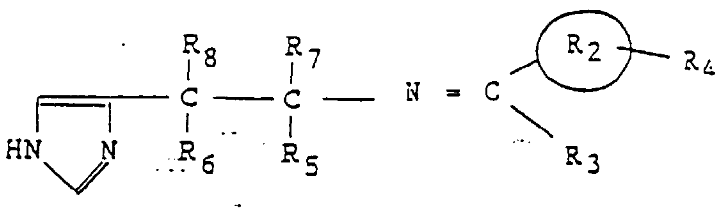

où $R_5$, $R_6$, $R_7$ et $R_8$ représentent chacun un atome d'hydrogène ou un groupe méthyle ou bien $R_5$ et $R_6$, pris ensemble, forment un groupe méthylène, $R_5$, $R_6$, $R_7$ et $R_8$ ne pouvant désigner simultanément l'hydrogène,

$R_2$ est un groupe aryle ou un groupe hétérocyclique éventuellement lié à $R_3$ et éventuellement mono-ou polysubstitué avec $R_4$,
$R_3$ a la signification de $R_2$ ou $R_4$ et
$R_4$ est l'hydrogène ou $OR_3$ ou $COOR_3$ ou un halogène ou $CF_3$ ou un alkyle non substitué, mono- ou polysubstitué ou l'un de ses sels pharmaceutiquement acceptables et un véhicule ou excipient pharmaceutiquement acceptable.

**Patentansprüche**

1. Von Histamin abgeleitete Verbindung mit einer starken Affinität für den $H_3$-Rezeptor von Histamin, dadurch gekennzeichnet, daß sie ein Azomethin der allgemeinen Formel ist:

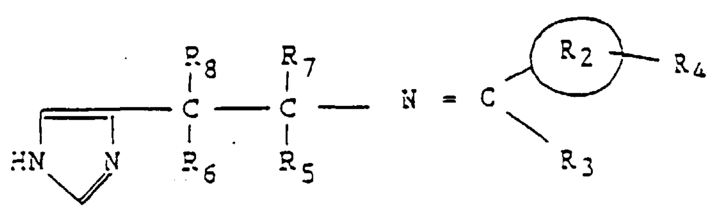

in der $R_5$, $R_6$, $R_7$ und $R_8$ jeweils ein Wasserstoffatom oder eine Methylgruppe darstellen oder $R_5$ und $R_6$ gemeinsam eine Methylengruppe bedeuten, wobei $R_5$, $R_6$, $R_7$ und $R_8$ nicht gleichzeitig Wasserstoff darstellen können,

$R_2$ eine Arylgruppe oder eine heterocyclische Gruppe bedeutet, die gegebenenfalls an die Gruppe $R_3$ gebunden ist und gegebenenfalls einfach oder mehrfach mit $R_4$ substituiert ist,
$R_3$ die Bedeutungen von $R_2$ oder $R_4$ besitzt und
$R_4$ Wasserstoff oder $OR_3$ oder $COOR_3$ oder ein Halogen oder $CF_3$ oder eine unbustituierte, einfach oder

mehrfach substituierte Alkylgruppe bedeutet oder eines ihrer pharmazeutisch annehmbaren Salze.

2. Verbindung nach Anspruch 1, dadurch gekennzeichnet, daß $R_5$ Methyl bedeutet und $R_6$, $R_7$ und $R_8$ jeweils ein Wasserstoffatom darstellen.

3. Verbindung nach Anspruch 1, dadurch gekennzeichnet, daß $R_5$ und $R_7$ jeweils Methyl bedeuten und $R_6$ und $R_7$ jeweils ein Wasserstoffatom darstellen.

4. Verbindung nach Anspruch 1, dadurch gekennzeichnet, daß $R_5$ und $R_8$ jeweils Methyl bedeuten und $R_6$ und $R_7$ jeweils ein Wasserstoffatom darstellen.

5. Verbindung nach Anspruch 1, dadurch gekennzeichnet, daß $R_2$ eine Phenylgruppe darstellt, die gegebenenfalls einfach oder mehrfach mit einer Gruppe $R_4$ substituiert ist, welche eine Hydroxylgruppe, eine Methoxygruppe, ein Halogenatom oder $COO\text{-}CH_3$ darstellt, und $R_3$ eine Phenylgruppe bedeutet.

6. Verbindung nach Anspruch 1, dadurch gekennzeichnet, daß $R_2$ eine Phenylgruppe darstellt, die gegebenenfalls einfach oder mehrfach mit einer Gruppe $R_4$ substituiert ist oder mehrfach mit einer Gruppe $R_4$ substituiert ist, welche eine Hydroxylgruppe, eine Methylgruppe, ein Halogenatom oder eine Dimethylaminogruppe darstellt, und $R_3$ ein Wasserstoffatom oder Methyl bedeutet.

7. Verbindung nach Anspruch 1, dadurch gekennzeichnet, daß $R_2$ eine Imidazolylgruppe und $R_3$ und $R_4$ Wasserstoffatome bedeuten.

8. Verwendung der Verbindung nach irgendeinem der Ansprüche 1 bis 7 für die Herstellung eines Arzneimittels zur Inhibierung der Synthese und der Freisetzung von Histamin oder anderen durch den $H_3$-Rezeptor gesteuerten Mediatoren, insbesondere mit sedierender Wirkung, zur Steuerung des Schlafes, mit antikonvulsiver Wirkung, Anti-Schwindelwirkung, antidepressiver Wirkung, antiallergischer Wirkung, Anti-Asthmawirkung, antipruriginöser Wirkung, antiinflammatorischer Wirkung, antisekretorischer Wirkung oder Anti-Geschwürwirkung.

9. Pharmazeutische Zubereitung zur Inhibierung der Synthese und der Freisetzung von durch $H_3$-Rezeptoren gesteuerten Mediatoren enthaltend eine Verbindung der allgemeinen Formel

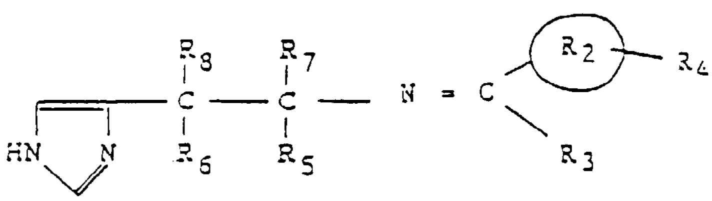

in der $R_5$, $R_6$, $R_7$ und $R_8$ jeweils ein Wasserstoffatom oder eine Methylgruppe darstellen oder $R_5$ und $R_6$ gemeinsam eine Methylengruppe bedeuten, wobei $R_5$, $R_6$, $R_7$ und $R_8$ nicht gleichzeitig Wasserstoff darstellen können,

$R_2$ eine Arylgruppe oder eine heterocyclische Gruppe bedeutet, die gegebenenfalls an die Gruppe $R_3$ gebunden ist und gegebenenfalls einfach oder mehrfach mit $R_4$ substituiert ist,
$R_3$ die Bedeutungen von $R_2$ oder $R_4$ besitzt und
$R_4$ Wasserstoff oder $OR_3$ oder $COOR_3$ oder ein Halogen oder $CF_3$ oder eine unsbustituierte, einfach oder mehrfach substituierte Alkylgruppe bedeutet oder eines ihrer pharmazeutisch annehmbaren Salze und ein pharmazeutisch annehmbares Trägermaterial oder Bindemittel.

**Claims**

1. Compound derived from histamine having a strong affinity for the $H_3$ receptor of histamine, characterised in that it consists of an azomethine of general formula

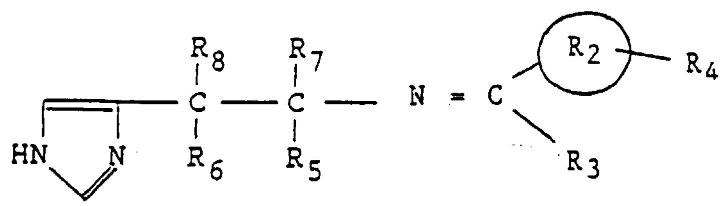

wherein $R_5$, $R_6$, $R_7$ and $R_8$ each represent a hydrogen atom or a methyl group or $R_5$ and $R_6$ taken together form a methylene group, whilst $R_5$, $R_6$, $R_7$ and $R_8$ cannot simultaneously denote hydrogen,

$R_2$ is an aryl group or a heterocyclic group optionally linked to $R_3$ and optionally mono- or polysubstituted with $R_4$,
$R_3$ has the same meaning as $R_2$ or $R_4$ and
$R_4$ is hydrogen or $OR_3$ or $COOR_3$ or a halogen or $CF_3$ or an unsubstituted, mono- or polysubstituted alkyl, or a pharmaceutically acceptable salt thereof.

**2.** Compound according to claim 1, characterised in that $R_5$ is methyl and $R_6$, $R_7$ and $R_8$ each represent a hydrogen atom.

**3.** Compound according to claim 1, characterised in that $R_5$ and $R_7$ each denote methyl and $R_6$ and $R_7$ each denote a hydrogen atom.

**4.** Compound according to claim 1, characterised in that $R_5$ and $R_8$ each denote methyl and $R_6$ and $R_7$ each denote a hydrogen atom.

**5.** Compound according to claim 1, characterised in that $R_2$ is a phenyl group optionally mono- or polysubstituted by a radical $R_4$ representing a hydroxy, a methoxy, a halogen or $COO-CH_3$ and $R_3$ represents a phenyl group.

**6.** Compound according to claim 1, characterised in that $R_2$ is a phenyl group optionally mono- or polysubstituted by a radical $R_4$ or polysubstituted by a radical $R_4$ denoting a hydroxy, a methyl, a halogen or a dimethylamino, and $R_3$ denotes a hydrogen atom or methyl.

**7.** Compound according to claim 1, characterised in that $R_2$ denotes an imidazolyl radical and $R_3$ and $R_4$ denote a hydrogen atom.

**8.** Use of the compound according to any one of claims 1 to 7 for preparing a pharmaceutical preparation inhibiting the synthesis and release of histamine or other mediators regulated by the $H_3$ receptor, notably with a sedative, sleep regulating, anticonvulsant, antivertiginous, antidepressant, antiallergic, antiasthmatic, antipruritic, anti-inflammatory, antisecretory or antiulcerative effects.

**9.** Pharmaceutical composition intended to inhibit the synthesis and release of the mediators regulated by the $H_3$ receptors containing a compound of general formula

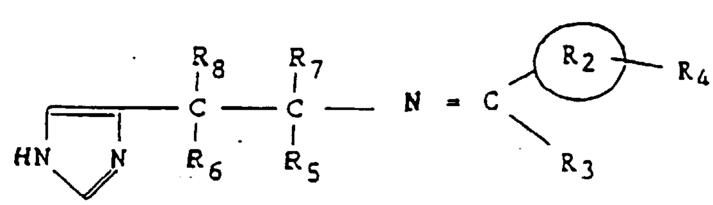

wherein $R_5$, $R_6$, $R_7$ and $R_8$ each represent a hydrogen atom or a methyl group or $R_5$ and $R_6$ taken together form a methylene group, whilst $R_5$, $R_6$, $R_7$ and $R_8$ cannot simultaneously denote hydrogen,

$R_2$ is an aryl group or a heterocyclic group optionally linked to $R_3$ and optionally mono- or polysubstituted with $R_4$,

$R_3$ has the same meaning as $R_2$ or $R_4$ and
$R_4$ is hydrogen or $OR_3$ or $COOR_3$ or a halogen or $CF_3$ or an unsubstituted, mono- or polysubstituted alkyl, or a pharmaceutically acceptable salt thereof, and a pharmaceutically acceptable carrier or excipient.